# EUROPEAN PATENT APPLICATION

(11) **EP 2 572 589 A1**
(43) Date of publication of application: **27.03.2013**
(21) Application number: 11783651.0
(22) Date of filing: 20.05.2011
(51) Int. Cl.: A23K 1/18, A23K 1/16

(54) **PIG FEED, AND METHOD FOR FEEDING SAME**

(30) Priority: 21.05.2010 JP 2010117569
(71) Applicant: Kaneka Corporation, Osaka (JP)
(72) Inventor: OHARA Takaaki, Tokyo 107-6025 (JP); TANI Shinichi, Tokyo 107-6025 (JP); HOSOE Kazunori, Osaka-shi Osaka 530-8288 (JP); UEMURA Tomohito, Osaka-shi Osaka 530-8288 (JP)
(74) Representative: Vossius & Partner
(86) International application number: PCT/JP2011/061635
(87) International publication number: WO 2011/145719

(57) **Abstract**

An object of the present invention is to provide a method for improving a farrowing rate of sows or improving growth and productivity of postnatal piglets, and a feed used in the method. The present invention relates to a method for improving a farrowing rate of a sow or improving growth and productivity of postnatal piglets, the method comprising administering coenzyme Q to a swine.

## Description

### TECHNICAL FIELD

The present invention relates to a method for improving a farrowing rate of sows or improving growth and productivity of postnatal piglets using a coenzyme Q, a feed used in the method, and a feeding regimen of the feed.

### BACKGROUND ART

Several methods for improving a farrowing rate of sows and methods for improving growth and productivity of postnatal piglets have been proposed. For example, Patent Literature 1 discloses a feed blend containing 0.001 to 0.1% by weight more L-tryptophan than a necessary amount which, when fed to mother sows, significantly reduces the numbers of stillbirths and premature piglets and significantly increases in the number of weaned piglets and the average weight of weaned piglets, and additionally shortens the interval of recurrent estrous of mother sows by about three days and improves estrous condition as well; Patent Literature 2 is concerned with an organic acid and/or an organic acid-containing product which, when fed to mother sows, help(s) steady growth of piglets, enhance(s) the rate of raising and the weight gain after weaning, and in turn, shorten (s) the term before shipping as well as the interval to the next recurrent estrus of the mother sows; Further, Patent Literature 3 relates to a feed containing an enzymatically decomposed guar gum which improves health of pregnant sows and survival and growth of newborn piglets; in addition, Patent Literature 4 is concerned with a feed containing a reduced form of folic acid which increases the level of active folic acid in plasma, and improves reproductive efficiency due to fertilization to deliver fetal from womb of mother sows.

On the other hand, coenzyme Q is an essential component found in living bodies of a broad range of species from bacteria to mammals and is known as a component of mitochondrial electron transport system in living cells. Coenzyme Q is known to have several physiological rolls such as activation of mitochondria to biosynthesize adenosine triphosphate (ATP), which is a biological energy source in living bodies, activation of cardiac function, stabilization effect on cell membranes, and protective effect on cells by its antioxidant activity.

Coenzyme Q is used for various applications. Specifically, an oxidized form of coenzyme Q10 is used, for example, in drugs for congestive heart failure because of its effect on heart. In addition to medical applications, it is used in oral nutrients and nutritional supplements like vitamins.

From 1960s, several proposals have been made concerning use of coenzyme Q as a component of feeds for economically useful animals such as livestock and poultry. For example, Patent Literature 5 is concerned with providing a feed containing coenzyme Q for animals, in particular for poultry and demonstrates that the feed increases the weight of chickens; Further, Patent Literature 6 states that coenzyme Q is useful as a feed additive; and Patent Literature 7 states that coenzyme Q is effective for broilers in preventing ascites, and specifically demonstrates that a feed or drink containing coenzyme Q9 at a concentration of 20 ppm or more reduces the frequency of ascites and increases a growth rate.

### CITATION LIST

### Patent Literature

Patent Literature 1: JP S58-201950 A
Patent Literature 2: JP H4-320652 A
Patent Literature 3: JP H7-8183 A
Patent Literature 4: JP H7-147911 A
Patent Literature 5: GB 918409 B
Patent Literature 6: JP S57-79891 A
Patent Literature 7: JP H6-287136 A

### SUMMARY OF INVENTION

### Technical Problem

The above-mentioned conventional techniques for improving a farrowing rate of sows and growth and productivity of postnatal piglets, however, do not satisfy all needs because of insufficient effects, lack of practicality, and the like, and thus are not perfect techniques that can be consistently used in actual swine farms. Accordingly, there is a strong need for techniques that ensure more piglets per mother sow in terms of the number of piglets survived or the number of weaned piglets, and certainly allow more piglets to survive until weaning.

An object of the present invention is to provide a method for improving a farrowing rate of sows or improving growth and productivity of postnatal piglets, and a feed used in the method.

### Solution to Problem

Examinations have been made to find a way to solve the above problem, and consequently, coenzyme Q was found to certainly improve not only a farrowing rate of sows but also growth and productivity of postnatal piglet when administered to the sows in certain periods.

Specifically, the present invention relates to a method for improving a farrowing rate of a sow or improving growth and productivity of postnatal piglets, characterized in that the method including administering coenzyme Q to a swine.

The present invention also relates to swinery method wherein the coenzyme Q is administered to a postnatal piglet or a mother sow.

Preferably, the coenzyme Q is administered to a mother sow.

Preferably, the coenzyme Q is administered to a mother sow during pregnancy.

Preferably, the coenzyme Q is administered to a mother sow during pregnancy at least for one month after mating.

Preferably, the coenzyme Q is administered to a mother sow at least for one month prior to parturition.

Preferably, the coenzyme Q is administered to a mother sow during lactation.

Preferably, the coenzyme Q is further administered to a postnatal piglet.

Preferably, the improvement in a farrowing rate of a sow is observed as an increase in fertility, a shortening of the interval of recurrent estrous, or an increase in the number of total births or the number of piglets survived.

Preferably, the improvement in growth and productivity of postnatal piglets is observed as an increase in the number of weaned piglets, an increase in weight gain, or an increase in the weight at weaning.

Preferably, a feed containing the coenzyme Q is administered to a mother sow.

Preferably, the coenzyme Q is derived from yeast.

The present invention also relates to a feed for a mother sow which contains coenzyme Q at a concentration of 20 ppm or more.

### Advantageous Effects of Invention

Although the number of still births and morality of postnatal piglets have been high due to high prolificity and immaturity of piglets, the present invention improves a farrowing rate of farmed sows and also improves the growth rate of postnatal piglets and the proportion of weaned piglets, and additionally improves the utilization frequency of mother sows. The method and the feed of the present invention contribute to improvement in productivity of swine, and to improvement in health of swine, thereby contributing also to improvement in animal welfare.

### DESCRIPTION OF EMBODIMENTS

The following is offered to illustrate the present invention in detail based on embodiments.

The present invention relates to a method for improving a farrowing rate of a sow or improving growth and productivity of postnatal piglets, which is characterized by administering coenzyme Q to a swine.

The "improvement in a farrowing rate of a sow" herein is observed as improvement in general farrowing factors related to mother sows such as the interval of recurrent estrous, fertility, the number of total births, lactation volume, and survival ratio of postnatal piglets (or the number of piglets survived). Specifically, the coenzyme Q is expected to, when administered to a mother sow, shorten the interval of recurrent estrous, improve the fertility (in particular, the fertility of second mating) of the mother sow, increase the number of total births, the number of piglets survived, the lactation volume, and the fertility without decreasing productivity, improve pregnancy cycles, reduce the number of albinism or mummified newborn piglets, and increase the weight of piglets at birth.

In addition, the "improvement in growth and productivity of postnatal piglets" herein is observed as improvement in growth and productivity factors related to postnatal piglets such as an increase in the numbers of milk-fed piglets and weaned piglets, a reduction in the number of damaged piglets after weaning and the number of dead piglets after weaning, an increase in feed conversion and weight gain which is accompanied with an increase in the weight at weaning, and a reduction in the frequency of diarrhea.

In the present invention, a target swine to which the coenzyme Q is administered is a swine that is an economically useful animal or an experimental animal reared by a person who utilizes products thereof. In the present invention, it is preferable to administer the coenzyme Q to a mother sow. The "mother sow" herein is intended to include any breeding sows which are kept to breed and have mated (copulated) at least once.

The administration period is not particularly limited, but is preferably a predetermined period prior to conception (mating) and a predetermined period during pregnancy, more preferably a predetermined period during pregnancy, and still more preferably after mating (early pregnancy) or late pregnancy. In the case that the coenzyme Q is administered prior to and/or after mating, the administration period may be, for example, started from at least three days prior to mating, preferably from one week prior to mating, and continued until at least one week after mating, preferably two weeks after mating, and more preferably one month after mating. Particularly, since the growth and the productivity of postnatal piglets, in particular, the number of weaned piglets are increased, the period during which the coenzyme Q is administered to a mother sow preferably covers one month after mating (i.e. early pregnancy period), and the length of this period in this case is more preferably not shorter than one month. In the case of administration in latter pregnancy period, the administration period may be, for example, from at least one month prior to parturition until parturition, preferably from two months prior to parturition until parturition, and more preferably three months prior to parturition until parturition. Preferably, the coenzyme Q is administered to a mother sow at least in middle or latter pregnancy period, in particular, from one month prior to parturition until parturition (i.e. latter pregnancy period) because the growth and the productivity of postnatal piglets, in particular, the weight at weaning are improved. In order to improve the fertility (in particular, the fertility of second mating) and to shorten the interval of recurrent estrous, the coenzyme Q is preferably administered to a mother sow for a period that includes a pregnancy period, a lactation period, and a period prior to mating.

In the present invention, as described in EXAMPLES below, the coenzyme Q, when administered to mother sows during pregnancy, surprisingly improves the growth and the productivity of piglets born to the mother sows even when it is not administered to the mother sows during lactation or the piglets. Needless to say, the coenzyme Q may be administered to mother sows during lactation or postnatal piglets. In the case that the coenzyme Q is administered to mother sows during lactation, the growth and the productivity of piglets which are fed with milk of the coenzyme Q-fed mother sows are also improved. In the case that the coenzyme Q is further administered to piglets of coenzyme Q-fed mother sows, the growth and the productivity of the piglets can be further improved. In this case, the administration period is not particularly limited, and may be a period prior to weaning (i.e. a milk feed period) or a period after weaning. The term "piglets" generally refers to swine that are up to three months of age after birth, but the coenzyme Q may be further administered to swine in a subsequent fattening period.

The coenzyme Q used in the present invention may be prepared in any form, and may be prepared as, for example, a chemically synthesized product, a fermentation product, an extract from a natural product, or the like. Specifically, a microorganism (e.g. yeast, bacterium) containing the coenzyme Q may be used as it is, or a crude purified product thereof or purified coenzyme Q from these may be used, for example. The degree of purification is also not limited. However, the amount of coenzyme Q administered should finally reach a predetermined concentration or higher. There are two forms of coenzyme Q: an oxidized form represented by the formula (1):

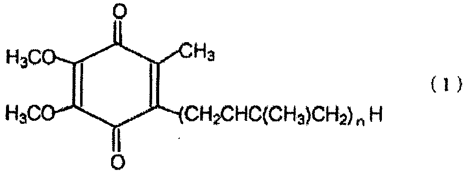

(wherein n is an integer of 1 to 12);
and a reduced form represented by the formula (2):

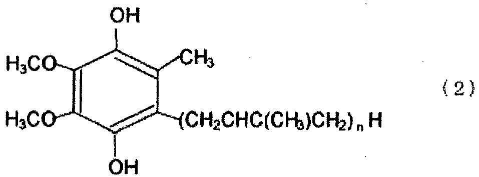

(wherein n is an integer of 1 to 12.)
The coenzyme Q used in the present invention may be in either of the oxidized form or the reduced form, or may be a mixture of these. In the case of a mixture, the ratio of these forms therein is not particularly limited. The coenzyme Q used in the present invention is preferably coenzyme Q10 (in the formulas (1) and (2), n is 10).

In the present invention, the coenzyme Q may be administered to a swine in any unlimited manner. The coenzyme Q may be mixed in a feed or drink, or may be directly administered to a swine in the drug formulation containing the coenzyme Q. In particular, in view of simplicity, the coenzyme Q is preferably mixed in a feed and the resulting feed is fed to a swine. A feed containing the coenzyme Q, in particular, a feed for mother sows or piglets which contains the coenzyme Q is also one aspect of the present invention.

Materials of swine feeds with which the coenzyme Q is mixed in the present invention are not particularly limited, and common materials may be used. Examples of such materials include grains (e.g. corn, milo, barley, wheat), chaff and bran (e.g. wheat bran), vegetable oil cakes (e.g. soy bean oil cake, rapeseed oil cake), animal feeds (e. g. fish meal, meat and bone meal), salt, oligosaccharides, silicic acids, various vitamins, minerals (e.g. calcium carbonate, dibasic calcium phosphate), amino acids, and organic acids. A commercial feed blend preliminarily containing these materials may be used.

The coenzyme Q may be added to these swine feed materials in any unlimited manner. The coenzyme Q-containing feed of the present invention may be prepared by directly adding the coenzyme Q to a feed material, or optionally preparing a feed additive or premix containing the coenzyme Q and then adding the feed additive or premix to a feed material. Examples of main components of such a feed additive or premix include, but are not limited to, probiotics (e.g. Enterococcus, Bacillus, Bifidus), antioxidants (e.g. ethoxyquin, dibutyl hydroxytoluene), fungicides (e.g. propionic acid, calcium propionate), binders (e.g. sodium alginate, sodium caseinate, carboxymethyl cellulose), emulsifiers (e.g. glycerin fatty acid esters, sucrose fatty acid esters, sorbitan fatty acid esters), amino acids (e.g. D,L-alanine, L-arginine, L-lysine hydrochloride), vitamins (e.g. L-ascorbic acid, β-carotene), minerals (e.g. potassium chloride, iron citrate, magnesium oxide), pigments (e.g. astaxanthin, canthaxanthin), and enzymes (e.g. amylase, phytase, lipase).

The coenzyme Q content in the feed of the present invention is not particularly limited, as long as provided the amount that produces the effects of the present invention. The content maybe, for example, not less than 10 ppm, preferably not less than 20 ppm, more preferably not less than 40 ppm, still more preferably not less than 60 ppm, and particularly preferably not less than 100 ppm. The upper limit thereof is not particularly limited. The upper limit is, however, preferably about 400 ppm for economic reasons, and about 300 ppm is enough to produce the effects. The content may be adjusted depending on the administration period and targets to be administered. For example, the coenzyme Q content in a feed to be administered in early pregnancy after mating and/or late pregnancy may be high (for example, not less than 60 ppm and preferably not less than 100 ppm) and a feed to be administered in a period other than these periods may be low (for example, about 20 ppm).

### EXAMPLES

The following examples are offered to demonstrate the present invention in more detail but the present invention should not be construed as being limited by the examples.
In each of the following examples, pure coenzyme Q10 derived from yeast (Kaneka Corp.) was used.

### (Example 1)

### (1) Test method

About 160 mother sows from an actual swine farm were used and the study group was fed with feeds (coenzyme Q content in all feeds: 60 ppm) which were prepared by mixing coenzyme Q10 in normal feeds for mother sows ("ecobreed" during pregnancy, "ecolitter" during lactation). This feed test continued for eight months in which feeding to all the mother sows was simultaneously started and simultaneously finished. Before and after the test period, only the original feeds for mother sows were fed. In this test of feeding to the sows at pregnancy period, as the pregnancy period of sows continues 114 days and the interval to the next conception after parturition is about 31 days, it is apparent that in this test, coenzyme Q10 was administered to the mother sows in various stages of pregnancy or lactation which include a specific period in latter pregnancy, the entire pregnancy period, periods prior to or after mating, and a period from mating to early pregnancy. The mother sows were reared and managed in a common manner.

In order to confirm that coenzyme Q10 was mixed in the feeds for mother sows, about 100 g of each coenzyme Q10-containing test feed were sampled from three points of different feeders after one week from the preparation thereof in the test period and the each sample was analyzed.
Additionally, in order to analyze the coenzyme Q10 content in milk of mother sows during lactation, about 15 ml of colostrum (first day) were sampled in a 50-ml disposal tube from six mother sows during lactation in the periods in which coenzyme Q10 was administered to them or the periods in which coenzyme Q10 was not administered to them, and the samples were analyzed for coenzyme Q10 and the ratio of the reduced form and the oxidized form of coenzyme Q10 by HPLC.

### (2) Test result

The HPLC analysis of coenzyme Q10 in the coenzyme Q10-containing feeds from the feeders in the test period revealed that the coenzyme Q10 content in the feeds was at least 40 ppm. On the other hand, the coenzyme Q10 content in the original feeds to which coenzyme Q10 was not added was found to be 0 ppm. These results concluded that coenzyme Q10 was certainly mixed in the feeds for mother sows which were used in the test periods.
The analytical results also revealed that the coenzyme Q10 content in colostrum from the mother sows in periods in which coenzyme Q10 was administered to them was apparently higher than that in periods in which coenzyme Q10 was not administered to them.
Compared to the periods in which coenzyme Q10 was not administered to the mother sows, it was confirmed that the number of total births was increased, the frequencies of albinism and mummified were decreased, and the numbers of milk-fed piglets and weaned piglets among piglets born to the mother sows were increased in the periods in which coenzyme Q10 was administered.
Additionally, piglets born to the mother sows under the test were found to have a heavier body weight as measured after three weeks from birth, and steadily grew with less episodes of diarrhea than piglets reared in a conventional manner.

### (Example 2)

The following example is concerned with an increase in productivity of piglets born to the coenzyme Q10-fed mother sows through the period of weaning, piglet and following fattening.

### (1) Test method

The piglets of the mother sows to which coenzyme Q10 had been administered in the test of Example 1 were fed with the following feeds: a feed prepared by adding coenzyme Q10 at about 80 ppm to a normal feed("Healthy Pig" (registered trademark)) for the weaning period; and feeds prepared by adding coenzyme Q10 at 40 ppm to normal feeds ("Take Five" for the former half of the piglet period, "Nobita kun" for the latter half of the piglet period, and "NIC Niku Buta EX" for the fattening period) . The piglets were reared and managed in a common manner. In all the periods, control groups were set up for each to which coenzyme Q10 was not administered, and the control group was also reared in a common manner.
The "weaning period" refers to the period from the 3rd day until 26th day after birth, and the "piglet period" refers to the period from the 27th day until the 84th day after birth.

### (2) Test result

The number of weaned piglets among coenzyme Q10-fed piglets per mother sow was larger than that of the control group.

### (Example 3)

Mother sows from an actual swine farm were used to evaluate how coenzyme Q10, when administered to the mother sows in middle or late pregnancy, effects on the increase in growth and productivity of postnatal piglets.

### (1) Test method

Test groups A to D were fed with feeds prepared by adding coenzyme Q10 at 100 ppm or 400 ppm to a normal feed for mother sows (crude protein 15.0% or more, crude fat 2.0% or more, crude fiber 5.0% or less, crude ash 8.0% or less, calcium 0.6% or more, phosphorus 0.4% or more, TDN 74.0% or more) in middle pregnancy (test groups A and B) or late pregnancy (test groups C and D), and were fed only with the normal feed for mother sows in the other periods. A control group was fed only with the normal feed for mother sows throughout the entire pregnancy period. Here, the term of "middle pregnancy" refers to the period from the 39th day until the 76th day after mating, and the term of "latter pregnancy" refers to the period (about 38 days) from the 77th day after mating until parturition. The test continued for about five and a half months. Also, coenzyme Q10 was not administered to the mother sows during lactation and the piglets born to the mother sows. As for head count of mother sows, each of control and test groups consisted of five mother sows per test, and the same test was repeated three times. Consequently, 15 sows in total were tested per a group. Primiparous sows were not used, and only multiparous sows (para 2 to 10) were used for the test. A coenzyme Q10-containing premix was prepared by adding coenzyme Q10 to defatted rice bran to be a concentration of 1/60, and was mixed with the normal feed with a measuring spoon (15 g/spoon) to the above-mentioned coenzyme Q10 concentrations. The feeds thus prepared were fed in a common manner. The average amount of the feed per mother sow was 2.5 kg/day in all the groups. The mother sows and the piglets born to the mother sows were reared in accordance with rearing management standards of the farm. The piglets born to the mother sows of the respective groups were evaluated for weight at birth, weight at weaning, and the like.

### (2) Test result

Table 1 shows the average weights of the piglets born to the mother sows of the each group at weaning respectively. The results show that coenzyme Q10 can, when administered at a concentration of 100 to 400 ppm to sows in the middle pregnancy period or the latter pregnancy period, certainly increase the weight at weaning, and further, this effect is particularly prominent when coenzyme Q10 is administered to sows in latter pregnancy period. Since the weight at birth was not so different between the test groups and the control group in this test, this test revealed a previously unknown, surprising effect that coenzyme Q10 can increase the weight of piglets at weaning even when their mother sows are fed with coenzyme Q10 only during pregnancy and reared under common conditions after parturition. Coenzyme Q10 is expected to produce a better effect when administered to mother sows throughout the entire pregnancy period not only in middle or latter pregnancy.

**[Table 1]**

| | Period of coenzyme Q10 administration | Amount of coenzyme Q10 (ppm) | Weight at weaning (average kg) |
|---|---|---|---|
| Control group | - | - | 7.89 |
| Test group A | Middle pregnancy | 100 | 8.37 |
| Test group B | Middle pregnancy | 400 | 8.26 |
| Test group C | Late pregnancy | 100 | 8.55 |
| Test group D | Late pregnancy | 400 | 8.51 |

### (Example 4)

Mother sows from an actual swine farm were used to evaluate how coenzyme Q10 effects on a farrowing rate of the mother sows and growth and productivity of postnatal piglets when administered to the mother sows in early, middle or latter pregnancy period.

### (1) Test method

Test groups E to G were fed with a feed prepared by adding coenzyme Q10 at a concentration of 100 ppm to a normal feed for mother sows (crude protein 15.0% or more, crude fat 2.0% or more, crude fiber 5.0% or less, crude ash 8.0% or less, calcium 0.6% or more, phosphorus 0.4% or more, TDN 74.0% or more) in early pregnancy period (test group E), middle pregnancy period (test group F) or latter pregnancy period (test group G), and were fed only with the normal feed for mother sows in the other periods. A control group was fed only with the normal feed for mother sows throughout the entire pregnancy period. The "early pregnancy" refers to the period from mating until the 38th day, the "middle pregnancy" refers to the period from the 39th day until the 76th day after mating, and the "latter pregnancy" refers to the period (about 38 days) from the 77th day after mating until parturition. Although whether sows are pregnant can be determined by sonography generally on the 25th or 26th day after mating, the test feeding was started without checking whether the mother sows were pregnant in this test. Sows that were found not to be pregnant later were eliminated from the test. Coenzyme Q10 was not administered to the mother sows during lactation and piglets born to the mother sows. Each of control and test groups consisted of five mother sows per test, and the same test was repeated three times. Consequently, 15 sows in total were tested per group. Primiparous sows were not used, and only multiparous sows (para 2 to 10) were used for the test. A coenzyme Q10-containing premix was prepared by adding coenzyme Q10 to defatted rice bran to a concentration of 1/60. This premix was added to the normal feed with a measuring spoon (15 g/spoon) to the above-mentioned coenzyme Q10 concentrations. The feeds thus prepared were fed in a common manner. The amount of the feed fed to each mother sow was 2.5 kg/day in average in all the groups. The mother sows and piglets born to the mother sows were reared in accordance with rearing management standards of the farm. The respective groups were evaluated based on the number of piglets (the number of total births) per mother sow, and the number of living piglets among them (the number of live births), and the number of piglets that survived to weaning (the number of weaned piglets).

### (2) Test result

Table 2 shows the numbers of weaned piglets of the respective groups. The results demonstrate that coenzyme Q10, when administered to sows during pregnancy, increases the number of weaned piglets, and that when coenzyme Q10 is administered to sows in early pregnancy, this effect is particularly prominent as evidenced by the increase in the average number of piglets per mother sow by not less than 1. An increase in the number of weaned piglets by 1 is a surprising effect which produces sufficient economic advantages in the swine farming industry. The group to which 100 ppm of coenzyme Q10 was administered in early pregnancy resulted in not only improvement in the growth and the productivity of postnatal piglets as evidenced by an increase in the number of weaned piglets, but also improvement in a farrowing rate as evidenced by an increase in the numbers of total births and live births by not less than 1 compared to the control group.

**[Table 2]**

| | Period of coenzyme Q10 administration | Amount of coenzyme Q10 (ppm) | Number of weaned piglets (average per mother sow) |
|---|---|---|---|
| Control group | - | - | 9.3 |
| Test group E | Early pregnancy | 100 | 10.4 |
| Test group F | Middle pregnancy | 100 | 9.6 |
| Test group G | Late pregnancy | 100 | 9.7 |

### (Example 5)

Mother sows from an actual swine farm were used to evaluate how administration of coenzyme Q10 acts on the fertility of second mating.

### (1) Test method

A coenzyme Q10-fed test group was fed with a feed prepared by adding coenzyme Q10 at a concentration of 100 ppm to a normal feed for mother sows (Kyodo Shiryo Co., Ltd.) for a test period (four months). A control group was fed with only the normal feed for mother sows. The sows were tested for pregnancy on the 28th day after mating, and unfertilized sows were mated again. The fertility was evaluated as a pregnancy rate for three months from one month after the start of the test until the end of the test. Whether the sows were pregnant (fertilized) was determined by sonography or the like on the 28th day after first mating and second mating. The test group consisted of 952 mother sows, and the control group consisted of 992 mother sows.

### (2) Test result

Whether the sows were pregnant was determined on the 28th day after the second mating, and the test and control groups were compared for the fertility of the sows by the second mating. Consequently, the fertilities of the test group and the control group were found to be 94% and 85%, respectively. Thus, administration of coenzyme Q10 was found to improve the fertility of second mating.

### (Example 6)

Mother sows were reared in an actual swine farm to evaluate whether administration of coenzyme Q10 shortens the interval of recurrent estrous (periods from the end of lactation until next estrus).

### (1) Test method

A coenzyme Q10-fed test group was fed with a feed prepared by adding coenzyme Q10 at a concentration of 100 ppm to a normal feed for mother sows (Kyodo Shiryo Co., Ltd.) for a test period (four months). A control group was fed with only the normal feed for mother sows. Estrous cycles were evaluated by observing whether sows were in estrus for three months from one month after the start of the test until the end of the test. The test group consisted of 1055 mother sows, and the control group consisted of 1022 sows.

### (2) Test result

The interval of recurrent estrous of the test group was found to be 5.59 days and the interval of recurrent estrous of the control group was found to be 5.92 days. Thus, administration of coenzyme Q10 was found to have an effect of shortening the interval of recurrent estrous by 0.33 days.

## Claims

1. A method for improving a farrowing rate of a sow or improving growth and productivity of postnatal piglets, **characterized in that** the method comprises administering coenzyme Q to a swine.

2. The method according to claim 1,
wherein the coenzyme Q is administered to a mother sow.

3. The method according to claim 2,
wherein the coenzyme Q is administered to a mother sow during pregnancy.

4. The method according to claim 2,
wherein the coenzyme Q is administered to a mother sow during pregnancy at least for one month after mating.

5. The method according to claim 2,
wherein the coenzyme Q is administered to a mother sow at least for one month prior to parturition.

6. The method according to claim 2,
wherein the coenzyme Q is administered to a mother sow during lactation.

7. The method according to any one of claims 2 to 6,
wherein the method further comprises administering the coenzyme Q to a postnatal piglet.

8. The method according to any one of claims 1 to 7,
wherein the improvement in a farrowing rate of a sow is observed as an increase in fertility, a shortening of the interval of recurrent estrous, or an increase in the number of total births or in the number of piglets survived.

9. The method according to any one of claims 1 to 7,
wherein the improvement in growth and productivity of postnatal piglets is observed as an increase in the number of weaned piglets, an increase in weight gain, or an increase in the weight at weaning.

10. The method according to any one of claims 1 to 9,
wherein a feed containing the coenzyme Q is administered to a mother sow.

11. The method according to any one of claims 1 to 10,
wherein the coenzyme Q is derived from yeast.

12. A feed for a mother sow, comprising coenzyme Q at a concentration of 20 ppm or more.
